# EUROPEAN PATENT APPLICATION

(11) **EP 4 620 506 A1**
(43) Date of publication of application: **24.09.2025**
(21) Application number: 23891354.5
(22) Date of filing: 31.10.2023
(51) Int. Cl.: A61M 25/09

(54) **MEDICAL DEVICE**

(30) Priority: 16.11.2022 JP 2022183146
(71) Applicant: ASAHI INTECC CO., LTD., Seto-shi, Aichi, 489-0071 (JP)
(72) Inventor: TAKAI, Ami, Seto-shi, Aichi 489-0071 (JP); NAGAO, Narumi, Seto-shi, Aichi 489-0071 (JP); USHIDA, Keisuke, Seto-shi, Aichi 489-0071 (JP); CHIKAOKA, Sora, Seto-shi, Aichi 489-0071 (JP)
(74) Representative: Winter, Brandl - Partnerschaft mbB
(86) International application number: PCT/JP2023/039253
(87) International publication number: WO 2024/106201

(57) **Abstract**

A guide wire 10 (medical device) includes a first region 10a having a first surface property, and a second region 10b having a second surface property and located on a proximal end side with respect to the first region. In the guide wire 10, the first surface property includes a property that a surface of the first region 10a has a first frictional resistance value in a friction and wear test, the second surface property includes a property that a surface of the second region 10b has a second frictional resistance value in the friction and wear test under the same conditions as for the surface of the first region 10a. The first frictional resistance value is higher than the second frictional resistance value. This guide wire 10 (medical device) makes it possible to control progression of prolapse in spite of a simple structure.

## Description

### TECHNICAL FIELD

The present disclosure relates to a medical device.

### BACKGROUND ART

Medical devices such as guide wires have been conventionally used for guiding, to a target site, a catheter-like medical instrument that is inserted into a tubular organ of a human body, such as a blood vessel or a digestive organ.

There is a procedure in which, when a device such as a catheter and an internal indwelling instrument is guided to a target site in a blood vessel using a medical device such as a guide wire, the guide wire is pushed forward in the blood vessel while the distal end of the guide wire is folded back and bent into a U-shape (i.e. a prolapsing state) (an increase in a progression length of the prolapse means that the prolapse progresses, in some cases).

Patent Literature 1 discloses a technique in which a rigidity difference is provided in a longitudinal direction of a guide wire so that the prolapse can be prevented from progressing to a rear end side with respect to the position of the rigidity difference. Specifically, the rigidity difference is provided in a longitudinal direction of the guide wire by factors that e.g. a coil surrounding the outer periphery of the core shaft has a double structure of an outer flexible tube body and an inner flexible tube body on the distal end portion of the guide wire, a distal end of the inner flexible tube body deviates to a proximal end side with respect to a distal end of the outer flexible tube body, a joint part having a high rigidity is partially formed, the inner flexible tube body is processed into a tapered shape with an outer diameter decreasing toward the distal end. That means, in Patent Literature 1, the progression length of the prolapse is controlled by utilizing the rigidity difference in the longitudinal direction of the guide wire.

### CITATION LIST

### Patent Literature

Patent Literature 1: JP 2012-055731 A

### SUMMARY OF INVENTION

### TECHNICAL PROBLEM

However, there have been problems that, when a rigidity difference is structurally provided as the guide wire disclosed in Patent Literature 1, the guide wire tends to break at a part of the rigidity difference, i.e. at a physical rigidity change point, and once the guide wire is bent, a remaining angle is generated, resulting in a lowered operability. Also, there have been problems that the structure causing such a rigidity difference is relatively complicated, and manufacturing burdens are high, such as increased numbers of components and processes and an increased assembly cost.

The present disclosure has been made in view of the above circumstances, and an object of the present disclosure is to provide a medical device capable of controlling progression of prolapse in spite of a simple structure.

### SOLUTION TO PROBLEM

In order to achieve the above object, the present disclosure provides a medical device including a first region having a first surface property, and a second region having a second surface property and located on a proximal end side with respect to the first region (Disclosure 1).

According to this disclosure (Disclosure 1), in spite of a simple structure, a high friction region and a low friction region can be provided on the distal end side and the proximal end side respectively by controlling the surface properties of the first region and the second region, so that progression of prolapse can be controlled.

In the above disclosure (Disclosure 1), the first surface property includes a property that the surface of the first region has a first frictional resistance value in a friction and wear test, and the second surface property includes a property that the surface of the second region has a second frictional resistance value in a friction and wear test under the same conditions as for the surface of the first region, in which the first frictional resistance value may be higher than the second frictional resistance value (Disclosure 2).

In the above disclosure (Disclosure 2), when the friction and wear test is conducted under a test load of 1.96 N and at a test rate of 120 mm/min, the first frictional resistance value may be 0.23 N or higher (Disclosure 3), the second frictional resistance value may be 0.18 N or lower (Disclosure 4), a difference between the first frictional resistance value and the second frictional resistance value may be 0. 04 N or larger (Disclosure 5), and a value obtained by dividing the first frictional resistance value by the second frictional resistance value may be 1.2 or higher (Disclosure 6).

In the above disclosure (Disclosure 2), when the friction and wear test is conducted under a test load of 1.47 N and at a test rate of 120 mm/min, the first frictional resistance value may be 0.17 N or higher (Disclosure 7), the second frictional resistance value may be 0.11 N or lower (Disclosure 8), a difference between the first frictional resistance value and the second frictional resistance value may be 0. 05 N or larger (Disclosure 9), and a value obtained by dividing the first frictional resistance value by the second frictional resistance value may be 1.5 or higher (Disclosure 10).

In the above disclosure (Disclosure 1), the first surface property includes a property that a surface friction coefficient of the first region is a first friction coefficient, the second surface property includes a property that a surface friction coefficient of the second region is a second friction coefficient, in which the first friction coefficient may be larger than the second friction coefficient (Disclosure 11).

In the above disclosure (Disclosure 11), the first friction coefficient may be 0.1 or larger (Disclosure 12), the second friction coefficient may be 0.09 or smaller (Disclosure 13), a difference between the first friction coefficient and the second friction coefficient may be 0.01 or larger (Disclosure 14), and a value obtained by dividing the first friction coefficient by the second friction coefficient may be 1.1 or higher (Disclosure 15).

In the above disclosure (Disclosure 1), the first surface property may include a property that the surface of the first region is hydrophobic (Disclosure 16) or a property that a silicone coating layer is formed on the surface of the first region (Disclosure 17), and the second surface property may include a property that the surface of the second region is hydrophilic (Disclosure 18) or a property that a hydrophilic coating layer is formed on the surface of the first region (Disclosure 19).

In the above disclosure (Disclosure 1), the first region may include a distal end of the medical device (Disclosure 20).

The above disclosures (Disclosures 1 to 20) may include an elongated core shaft and a cylindrical body provided on an outside of the core shaft, in which an outer peripheral surface of the cylindrical body may be partitioned into a first region located on the distal end side and a second region located on the proximal end side with respect to the first region (Disclosure 21).

### BRIEF DESCRIPTION OF DRAWINGS

FIG. 1 is an explanatory diagram illustrating a structure of a guide wire according to an embodiment of the present disclosure.
FIG. 2 is an explanatory diagram illustrating a structure of a guide wire in a modification.
FIG. 3 is an explanatory diagram illustrating a structure of a sample guide wire used for a surface frictional resistance value measurement test.
FIG. 4 is an explanatory diagram illustrating a configuration of a measuring equipment used for the surface frictional resistance value measurement test.
FIG. 5 is an explanatory schematic diagram illustrating points at which a sample is measured.
FIG. 6 is an explanatory schematic diagram illustrating a blood vessel model used for a prolapse test.
FIG. 7 is an explanatory diagram illustrating a state that a distal end of the guide wire is intentionally hooked at a lateral branch in the prolapse test.
FIG. 8 is an explanatory diagram illustrating a state that the distal end of the guide wire enters the lateral branch after the state of FIG. 6 in the prolapse test.
FIG. 9 is an explanatory diagram illustrating a state that the guide wire does not enter the lateral branch but prolapses and progresses in a main vessel after the state of FIG. 6 in the prolapse test.
FIG. 10 is an enlarged view of a distal end portion of the guide wire illustrated in FIG. 9.

### EMBODIMENTS OF THE INVENTION

A guide wire 10 according to embodiments of the present disclosure will be explained below with reference to the drawings. The guide wire 10 is a medical device, which is used for inserting a catheter into a blood vessel, a digestive organ, or the like. A distal end side of the guide wire 10 is a side to be inserted into a body, and a proximal end side of the guide wire 10 is a side to be operated by a professional such as a surgeon. The present disclosure is not limited to the embodiments explained below, and the described embodiments are merely examples for explaining the technical features of the present disclosure. The shapes and dimensions illustrated in the drawings are merely illustrated to facilitate understanding of the contents of the present disclosure, and do not accurately reflect actual shapes and dimensions.

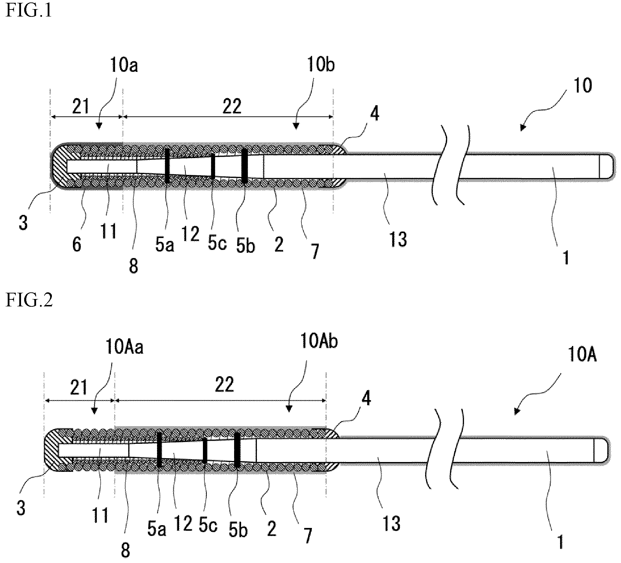

In this specification, the "distal end side" means a direction along an axial direction of the guide wire, in which the guide wire progresses toward a target site. The "proximal end side" means a direction along the axial direction of the guide wire, which is opposite to the direction toward the distal end side. The "distal end" refers to an end portion on a distal end side of any member or site, and the "proximal end" refers to an end portion on a proximal end side of any member or site. The "distal end portion" refers to, in any member or site, a part including the distal end thereof and extending from the distal end toward the proximal end side up to the middle of the member or the like, and the "proximal end portion" refers to, in any member or site, a part including the proximal end thereof and extending from the proximal end toward the distal end side up to the middle of the member or the like. In FIG. 1, the left side in the drawing is the "distal end side" to be inserted into a body, and the right side in the drawing is the "proximal end side" to be operated by a professional.

FIG. 1 is an explanatory diagram illustrating a structure of the guide wire 10 according to the present embodiments. The guide wire 10 includes an elongated core shaft 1 and a cylindrical body 2 provided outside the core shaft 1. A distal tip 3 for joining the core shaft 1 and the cylindrical body 2 is provided on the distal end of the guide wire 10, and a fixation portion 4 for fixing the core shaft 1 and the cylindrical body 2 is provided on a proximal end of the cylindrical body 2. An inner cylindrical body 8 is arranged along an outer periphery of the core shaft 1 on the inside of the cylindrical body 2.

The core shaft 1 is an elongated member serving as a shaft of the guide wire 10. As illustrated in FIG. 1, the core shaft 1 has a small diameter portion 11 on the distal end side and a large diameter portion 13 on the proximal end side, and has a tapered portion 12 disposed between the small diameter portion 11 and the large diameter portion 13 and having an outer diameter gradually decreasing from the proximal end side toward the distal end side. The core shaft 1 can be formed of, for example, materials such as stainless alloys (SUS302, SUS304, SUS316, etc.), superelastic alloys such as an Ni-Ti alloy, a piano wire, a nickel-chromium alloy, a cobalt alloy, and tungsten. The material for the core shaft 1 is not limited thereto, and the core shaft 1 may be formed of other known materials as long as the core shaft 1 itself can be prevented from being cut and has a rotatable distal end portion.

The small diameter portion 11 has a columnar shape with an outer diameter constant from the distal end to the proximal end. Alternatively, the small diameter portion 11 may be formed so as to have a flat cross-sectional shape by press working. The large diameter portion 13 has a columnar shape with an outer diameter constant from the distal end to the proximal end. The tapered portion 12 has a circular truncated cone shape with an outer diameter gradually increasing from the distal end toward the proximal end, and also has a sectional secondary moment gradually increasing from the distal end toward the proximal end.

The cylindrical body 2 is wound around the core shaft 1 so as to cover the outer peripheries of the small diameter portion 11, the tapered portion 12, and a part of the large diameter portion 13 of the core shaft 1.

The cylindrical body 2 may be a single coil that is formed into a hollow cylindrical shape by spirally winding one wire having a circular cross-section, or may be a hollow twisted wire coil that is formed into a hollow cylindrical shape using a twisted wire with a plurality of wires twisted. The cylindrical body 2 may be configured by combining a single coil and a hollow twisted wire coil. The cylindrical body 2 can be formed of, for example, stainless alloys (SUS302, SUS304, SUS316, etc.), superelastic alloys such as an Ni-Ti alloy, a piano wire, a nickel-chromium alloy, radiotransparent alloys such as a cobalt alloy, gold, platinum, tungsten, and radiopaque alloys such as an alloy containing these elements (e.g. a platinum-nickel alloy). The material for the cylindrical body 2 is not limited thereto, and may be formed of known materials other than the above-described materials. In the present embodiment, the cylindrical body 2 has a distal end side formed of a radiopaque alloy and a proximal end side formed of a stainless alloy, and is configured so as to have an outer diameter substantially constant from the distal end to the proximal end.

The distal tip 3 for joining the core shaft 1 with the cylindrical body 2 is formed on the distal end of the guide wire 10 (i.e. the distal end of the core shaft 1). The distal tip 3 is formed of a metal solder such as silver braze, gold braze, zinc, an Sn-Ag alloy, and an Au-Sn alloy, and the distal end of the core shaft 1 and the distal end of the cylindrical body 2 are firmly fixed to each other by the metal solder. The distal tip 3 may be formed of an adhesive such as an epoxy adhesive so that the distal end of the core shaft 1 and the distal end of the cylindrical body 2 are firmly fixed to each other by the adhesive.

The fixation portion 4 for fixing the core shaft 1 with the cylindrical body 2 is formed on the proximal end of the cylindrical body 2. The fixation portion 4 is formed of a metal solder such as silver braze, gold braze, zinc, an Sn-Ag alloy, and an Au-Sn alloy, and the proximal end of the cylindrical body 2 is firmly fixed to the large diameter portion 13 of the core shaft 1 by the metal solder. The fixation portion 4 may be formed of an adhesive such as an epoxy adhesive so that the large diameter portion 13 of the core shaft 1 and the proximal end of the cylindrical body 2 are firmly fixed to each other by the adhesive.

Two joint parts 5a and 5b for joining the tapered portion 12 of the core shaft 1 with the cylindrical body 2 are formed inside the cylindrical body 2. The joint parts 5a and 5b are formed of a metal solder such as silver braze, gold braze, zinc, an Sn-Ag alloy, and an Au-Sn alloy, and the tapered portion 12 of the core shaft 1 and the cylindrical body 2 are firmly fixed to each other by the metal solder. The joint parts 5a and 5b may be formed of an adhesive such as an epoxy adhesive so that the tapered portion 12 of the core shaft 1 and the cylindrical body 2 are firmly fixed to each other by the adhesive.

The inner cylindrical body 8 is shorter than the cylindrical body 2, and is wound on the outside of the core shaft 1 so as to cover the outer periphery of the core shaft 1 ranging from the small diameter portion 11 to a part of the tapered portion 12. Accordingly, only on the distal end portion of the guide wire 10, the cylindrical body 2 and the inner cylindrical body 8 overlap with each other on the outside of the core shaft 1.

The inner cylindrical body 8 has a distal end firmly fixed to the distal tip 3, and a proximal end firmly fixed to the tapered portion 12 of the core shaft 1 by a joint part 5c. The joint part 5c may be formed of a metal solder such as silver braze, gold braze, zinc, an Sn-Ag alloy, and an Au-Sn alloy, or an adhesive such as an epoxy adhesive.

The inner cylindrical body 8 may be a single coil that is formed into a hollow cylindrical shape by spirally winding one wire having a circular cross-section, or may be a hollow twisted wire coil that is formed into a hollow cylindrical shape using a twisted wire with a plurality of wires twisted. The inner cylindrical body 8 may be configured by combining a single coil and a hollow twisted wire coil. The inner cylindrical body 8 can be formed of, for example, stainless alloys (SUS302, SUS304, SUS316, etc.), superelastic alloys such as an Ni-Ti alloy, a piano wire, a nickel-chromium alloy, radiotransparent alloys such as a cobalt alloy, gold, platinum, tungsten, and radiopaque alloys such as an alloy containing these elements (e.g. a platinum-nickel alloy). The material for the inner cylindrical body 8 is not limited thereto, and may be formed of known materials other than the above-described materials. In this modification, the entire inner cylindrical body 8 is formed as a single member wholly made of same materials, and configured to have an outer diameter constant from the distal end to the proximal end.

An outer peripheral surface of the cylindrical body 2 is partitioned into a first surface region 21 located on the distal end side and a second surface region 22 located on the proximal end side with respect to the first surface region 21, and a surface property of the first surface region 21 is different from a surface property of the second surface region 22. Since the cylindrical body 2 having the first surface region 21 and the second surface region 22 on the outer peripheral surface is provided on the outside of the core shaft 1, the guide wire 10 includes a first region 10a having a first surface property and a second region 10b having a second surface property and located on the proximal end side with respect to the first region 10a. The first region 10a of the guide wire 10 includes the distal end of the guide wire 10.

The first surface property of the first region 10a means a property that, when the first region 10a is subjected to a friction and wear test, the first region 10a has a first frictional resistance value R₁. The second surface property of the second region 10b means a property that, when the second region 10b is subjected to the friction and wear test under the same condition as for the first region 10a, the second region 10b has a second frictional resistance value R₂. In the guide wire 10, the first frictional resistance value R₁ is higher than the second frictional resistance value R₂. By controlling the surface property of the first region 10a and the surface property of the second region 10b in this way, the guide wire 10 can have a high friction region on the distal end side and a low friction region on the proximal end side, so that progression of the prolapse can be controlled.

Specifically, when a friction and wear test is conducted under a test load of 1.96 N and at a test rate of 120 mm/min using a known friction and wear testing machine, the first frictional resistance value R₁ is preferably 0.23 N or higher. In the process of inserting the guide wire 10 into a blood vessel, prolapse is initiated when the distal end portion (the first region 10a) of the guide wire 10 is hooked at a part branching from a main vessel to a lateral branch in the blood vessel, a blood vessel wall or the like in the blood vessel. In the process of inserting the guide wire 10 into the blood vessel, when the first frictional resistance value R₁ is 0.23 N or higher, the distal end portion of the guide wire 10 is hooked at a part branching from a main vessel to a lateral branch in the blood vessel, or a blood vessel wall or the like in the blood vessel, and thereby the prolapse is triggered. In the process of inserting the prolapsing guide wire 10 into the blood vessel, when the first frictional resistance value R₁ is 0.23 N or higher, the progress length of the prolapse is restored to a proper length by pulling the guide wire 10 in the proximal end direction even if the prolapse excessively progresses.

When a friction and wear test is conducted under a test load of 1.96 N and at a test rate of 120 mm/min using a known friction and wear testing machine, the second frictional resistance value R₂ is preferably 0.18 N or lower. In the process of inserting the guide wire 10 into the blood vessel while the distal end portion of the guide wire 10 prolapses, when the second frictional resistance value R₂ is 0.18 N or lower, any friction sufficient to advance the prolapse is not generated between the second region 10b of the guide wire and the blood vessel wall or the like in the blood vessel, so that the progression of the prolapse is terminated at an appropriate position. Since a boundary between the first region 10a (first surface region 21) and the second region 10b (second surface region 22) serves as a reference position for determining to what extent the prolapse is allowed to progress in the guide wire 10, the lengths of the first region 10a and the second region 10b can be arbitrarily determined depending on the intended purpose of the guide wire 10, or the like.

In particular, when a friction and wear test is conducted under a test load of 1.96 N and at a test rate of 120 mm/min using a known friction and wear testing machine, a difference between the first frictional resistance value R₁ and the second frictional resistance value R₂ is preferably 0.04 N or larger, and a value obtained by dividing the first frictional resistance value R₁ by the second frictional resistance value R₂ is preferably 1.2 or higher. The structure including the first region 10a and the second region 10b having such surface properties makes it possible to achieve the guide wire 10 with an appropriately controlled prolapse progression length.

Alternatively, when a friction and wear test is conducted under a test load of 1.47 N and at a test rate of 120 mm/min using a known friction and wear testing machine, the first frictional resistance value R₁ is preferably 0.17 N or higher. In the process of inserting the guide wire 10 into a blood vessel, prolapse is initiated when the distal end portion (the first region 10a) of the guide wire 10 is hooked at a part branching from a main vessel to a lateral branch in the blood vessel, or a blood vessel wall or the like in the blood vessel. In the process of inserting the guide wire 10 into the blood vessel, when the first frictional resistance value R₁ is 0.17 N or higher, the distal end portion of the guide wire 10 is hooked at a part branching from a main vessel to a lateral branch in the blood vessel, or a blood vessel wall or the like in the blood vessel, and thereby the prolapse is triggered. In the progress of inserting the prolapsing guide wire 10 into the blood vessel, when the first frictional resistance value R₁ is 0.17 N or higher, the progression length of the prolapse is restored to a proper length by pulling the guide wire 10 in the proximal end direction even if the prolapse excessively progresses.

When a friction and wear test is conducted under a test load of 1.47 N and at a test rate of 120 mm/min using a known friction and wear testing machine, the second frictional resistance value R₂ is preferably 0.11 N or lower. In the process of the guide wire 10 into the blood vessel while the distal end portion of the guide wire 1 prolapses, when the second frictional resistance value R₂ is 0.11 N or lower, any friction sufficient to advance the prolapse is not generated between the second region 10b of the guide wire and the blood vessel wall or the like in the blood vessel, so that the progression of the prolapse is terminated at an appropriate position.

In particular, when a friction and wear test is conducted under a test load of 1.47 N and at a test rate of 120 mm/min using a known friction and wear testing machine, a difference between the first frictional resistance value R₁ and the second frictional resistance value R₂ is preferably 0.05 N or larger, and a value obtained by dividing the first frictional resistance value R₁ by the second frictional resistance value R₂ is preferably 1.5 or higher. The structure including the first region 10a and the second region 10b having such surface properties makes it possible to achieve the guide wire 10 with an appropriately controlled prolapse progression length.

In the present embodiments, the first surface property of the first region 10a (first surface region 21) may be a property that a surface friction coefficient of the first region 10a is a first friction coefficient µ₁. The second surface property of the second region 10b (second surface region 22) may be a property that a surface friction coefficient of the second region 10b is a second friction coefficient µ₂. In this case, the first friction coefficient µ₁ is larger than the second friction coefficient µ₂ in the guide wire 10. By controlling the surface property of the first region 10a and the surface property of the second region 10b in this way, the guide wire 10 can have a high friction region on the distal end side and a low friction region on the proximal end side, so that progression of the prolapse can be controlled.

Specifically, the first friction coefficient µ₁ is preferably 0.1 or larger, and the second friction coefficient µ₂ is preferably 0.09 or smaller. The structure including the first region 10a and the second region 10b having such surface properties makes it possible to achieve the guide wire 10 with an appropriately controlled prolapse progression length.

In particular, the difference between the first friction coefficient µ₁ and the second friction coefficient µ₂ is preferably 0.01 or larger, and the value obtained by dividing the first friction coefficient µ₁ by the second friction coefficient µ₂ is preferably 1.1 or higher. The structure including the first region 10a and the second region 10b having such surface properties makes it possible to achieve the guide wire 10 with an appropriately controlled prolapse progression length.

The relationship between the first friction coefficient µ₁ as the surface friction coefficient of the first region 10a and the second friction coefficient µ₂ as the surface friction coefficient of the second region 10b may be adjusted e.g. by forming covering layers made of different materials on the both regions, or by forming a covering layer only on one region, or by performing different surface treatments on the both regions, or by forming covering layers made of the same material on the both regions and then performing a surface treatment only on a covering layer formed on one region.

In the present embodiments, the guide wire 10 has a first covering layer 6 formed on the surface of the first region 10a, and a second covering layer 7 formed on the second region 10b. Materials for the first covering layer 6 and the second covering layer 7 are selected so that the surface friction coefficient µ₁ of the first covering layer 6 is larger than the surface friction coefficient µ₂ of the second covering layer 7.

The first covering layer 6 is a silicone coating layer, and can be formed by a known coating forming method, e.g. by applying a medical-grade silicone solution to the surfaces of the distal end side of the cylindrical body 2 and the distal tip 3. That means, in the present embodiments, the first surface property of the first region 10a is surface hydrophobicity of the first region 10a, i.e. means that a silicone coating layer as the first covering layer 6 is formed on the surface of the first region 10a.

The second covering layer 7 is a hydrophilic coating layer and can be formed e.g. by a known coating forming method, in which a solution including a nonionic hydrophilic polymer such as polyvinyl alcohol, polyvinylpyrrolidone, polyethylene glycol, polyacrylamide, polymethylacrylamide, poly(2-hydroxyethyl methacrylate), and poly(N-hydroxyethylacrylamide); an anionic hydrophilic polymer such as polyacrylic acid, polymethacrylic acid, polymaleic acid, carboxymethyl cellulose, hyaluronic acid, and poly(2-acrylamide-2-methylpropanesulfonic acid); or a cationic hydrophilic polymer such as polyethyleneimine, polyallylamine, and polyvinylamine is applied to the proximal end-side surface of the cylindrical body 2, the surface of the fixation portion 4, and the proximal end-side surface of the large diameter portion 13 of the core shaft 1. That means, in the present embodiments, the second surface property of the second region 10b is surface hydrophilicity of the second region 10b, i.e. means that a hydrophilic coating layer as the second covering layer 7 is formed on the surface of the second region 10b.

According to this guide wire 10, by providing the first region 10a as the high friction region on the distal end side and the second region 10b as the low friction region on the proximal end side, the guide wire 10 becomes easier to bend up to the vicinity of the boundary between the first region 10a and the second region 10b to achieve a guide wire with the prolapse suppressed from progressing toward the proximal end side with respect to the vicinity or the boundary. In other words, on the surface of the cylindrical body 2 provided outside the core shaft 1 of the guide wire 10, the first surface region 21 as the high friction region is provided on the distal end side and the second surface region 22 as the low friction region is provided on the proximal end side, so that the guide wire 10 becomes easier to bend up to the vicinity of the boundary between the first surface region 21 and the second surface region 22 to achieve a guide wire with the prolapse suppressed from progressing toward the proximal end side with respect to the vicinity or the boundary. Since this guide wire 10 makes it possible to arbitrarily decide how to arrange the first surface region 21 and the second surface region 22 on the surface of the cylindrical body 2, there is no longer any need to make the structure of the guide wire complex as in the past to generate a rigidity gap, and therefore the progression of the prolapse can be suppressed at any position in spite of the simple structure. In particular, since the surface friction coefficient can be easily changed depending on the state and the type of the coating in each region, the degree of freedom for designing the guide wire can be increased.

The guide wire as a medical device according to the present disclosure has been explained above with reference to the drawings. The present disclosure is not limited to the above embodiments and can be variously modified. For example, in the above embodiments, the case of the core shaft having the large diameter portion on the proximal end side, the small diameter portion on the distal end side, and the tapered portion between the large diameter portion and the small diameter portion, has been explained as an example. However, the shape of the core shaft is not limited thereto.

### <Modifications>

A modification of the guide wire explained in the above embodiments will be explained below with reference to FIG. 2. In a guide wire 10A as a modification, the same components as for the guide wire 10 described above are marked with the same symbols as for the guide wire 10, and their descriptions are omitted. FIG. 2 is an explanatory diagram illustrating a structure of a modification of the guide wire according to the present disclosure, and the guide wire 10A in the modification is different from the above-described guide wire 10 in that the guide wire 10A does not have the silicone coating layer (first covering layer 6) covering the first region 10a.

In the present modification, the outer peripheral surface of the cylindrical body 2 is partitioned into the first surface region 21 located on the distal end side and the second surface region 22 located on the proximal end side with respect to the first surface region 21, and the surface property of the first surface region 21 differs from that of the second surface region. The cylindrical body 2 with the outer peripheral surface having the first surface region 21 and the second surface region 22 is disposed on the outside of the core shaft 1, so that the guide wire 10A has a first region 10Aa having the first surface property. Furthermore, the guide wire 10A has a second region 10Ab having the second surface property and located on the proximal end side with respect to the first region 10Aa. The first region 10Aa of the guide wire 10A includes the distal end of the guide wire 10A.

Even if the first region 10Aa is not covered, as long as the first region 10Aa has a surface friction coefficient larger than that of the second region 10Ab covered with the second covering layer 7, the guide wire 10A can easily prolapse up to the vicinity of the boundary between the first region 10Aa and the second region 10Ab to achieve a guide wire with the prolapse suppressed from progressing toward the proximal end side with respect to the vicinity of the boundary.

### [EXAMPLES]

The present disclosure will be explained below in more detail with reference to Examples and the like. The scope of the present disclosure is not limited to these Examples.

### <Measurement Test of Surface Frictional Resistance Value>

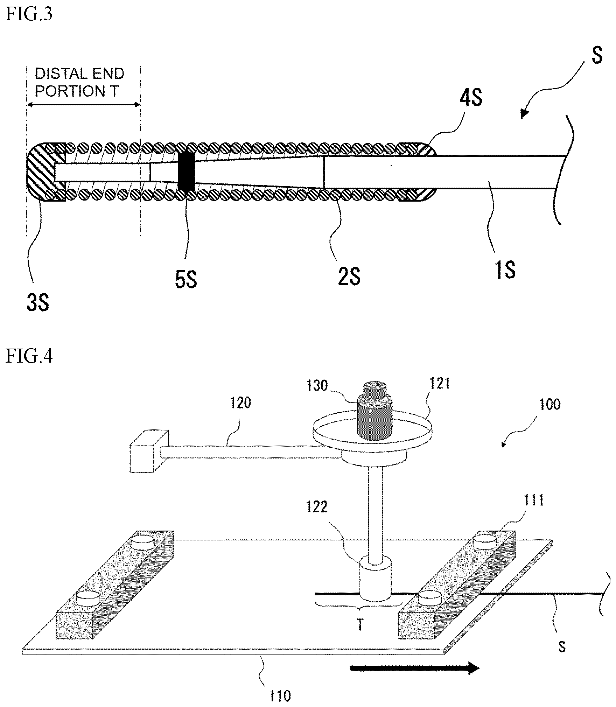

Different covering layers were formed on a distal end portion T of a sample guide wire S illustrated in FIG. 3 to prepare samples, and these samples were subjected to a friction and wear test to measure frictional resistance values of the surface of the distal end portion T.

### (Sample Guide Wire)

The sample guide wire S has a core shaft 1S made of a stainless steel alloy and having the same shape as the core shaft used for the above-described guide wires 10 and 10A, and is wound with a single coil 2S formed in a hollow cylindrical shape with a spirally wound wire having a circular cross-section so as to cover the outer periphery of a part of a small diameter portion, a tapered portion, and a part of a large diameter portion of the core shaft 1S. A distal tip 3S (silver braze) for joining the core shaft 1S with the single coil 2S is formed on the distal end of the sample guide wire S (i.e. distal end of the core shaft 1S), and a fixation portion 4S (silver braze) for fixing the core shaft 1S and the single coil 2S is formed on the proximal end of the single coil 2S. A joint part 5S (silver braze) for joining the tapered portion of the core shaft 1S and the single coil 2S is formed inside the single coil 2S. The distal end portion T of the sample guide wire S has a structure in which a pitch of the single coil 2S is slightly widened over a length of about 15 mm from the frontmost end.

### (Sample)

Different covering layers were formed on the distal end portion T of the sample guide wire S to prepare samples S1 to S5. The samples S1 to S3 have a hydrophilic coating layer on the distal end portion T, the sample S4 has a silicone coating layer on the distal end portion T, and the sample S5 has no coating layer on the distal end portion T. The conditions of the prepared samples S1 to S5 are presented in Table 1.

**[Table 1]**

| | Coating state of distal end portion T |
|---|---|
| Sample S1 | Coating with a hydrophilic coating liquid (film thickness: large) |
| Sample S2 | Coating with a hydrophilic coating liquid (film thickness: medium) |
| Sample S3 | Coating with a hydrophilic coating liquid (film thickness: small) |
| Sample S4 | Coating with a silicone coating agent |
| Sample S5 | No coating |

### (Friction and Wear Test)

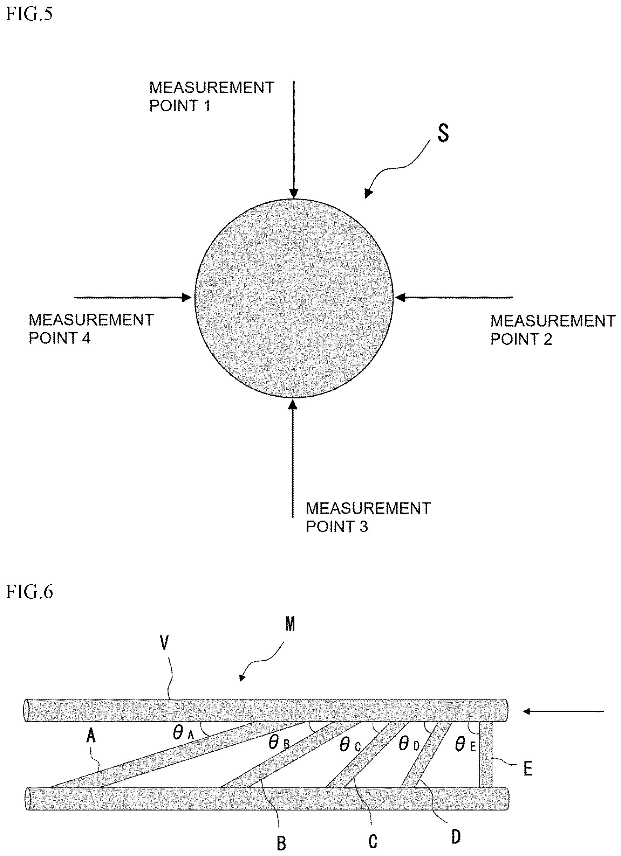

The frictional resistance value in this test is a dynamic frictional resistance value. For the friction and wear test, a friction and wear testing machine TYPE: 38 manufactured by Shinto Scientific Co., Ltd. was used. FIG. 4 illustrates a structure of a friction and wear testing machine 100. The friction and wear testing machine 100 is configured such that the sample guide wire S (samples S1 to S5) can be fixed onto a horizontally-movable measurement table 110 using a sample fixture 111, and when a weight 130 is placed on a weight pan 121 attached to an end portion of an arm 120, a measurement indenter 122 attached to the weight pan 121 is pressed against the sample guide wire S by the weight of the weight 130. In this test, a urethane sheet (thickness: 0.5 mm, hardness: Shore A90) was bonded to the bottom surface of the almost columnar measurement indenter 122, and the bottom surface of the measurement indenter 122 was brought into contact with a distal end portion T of each of the samples S1 to S5, and a frictional resistance generated by moving the measurement table 110 to the right side was measured by the measurement indenter 122. The friction and wear test was conducted in two patterns of test loads of 1.96 N (200 gf) and 1.47 N (150 gf), and at a test rate of 120 mm/min. Specifically, as illustrated in FIG. 5, the test was conducted from four sides: up, down, left, and right sides, for each of the samples S1 to S5, and a frictional resistance value was measured on each measurement point under a condition of a measurement length (distance in the longitudinal direction) of 10 mm and a measurement time of 5 seconds. The frictional resistance value was determined as a value excluding effects of a static frictional resistance. Specifically, a resistance value measured during 1 to 5 seconds, from the measurement time of 0 to 5, is adopted, and the average thereof is set as a frictional resistance value of respective distal end portions of the samples S1 to S5. FIG. 5 is a schematic diagram illustrating measurement directions of the samples S1 to A5 (where the measurement points were set on the samples S1 to S5) viewed in the transverse sectional direction of each of the samples S1 to S5.

Regarding the measurement results of the frictional resistance values on the distal end portions T of the samples S1 to S5, results of tests under the test load of 1.96 N (200 gf) are presented in Table 2, and results of tests under the test load of 1.47 N (150 gf) are presented in Table 3.

**[Table 2]**

| | Frictional resistance value of distal end portion T | |
|---|---|---|
| | (gf) | (N) |
| Sample S1 | 8.8 | 0.09 |
| Sample S2 | 11.2 | 0.11 |
| Sample S3 | 18.3 | 0.18 |
| Sample S4 | 23.4 | 0.23 |
| Sample S5 | 52.9 | 0.52 |

**[Table 3]**

| | Frictional resistance value of distal end portion T | |
|---|---|---|
| | (gf) | (N) |
| Sample S1 | 8.8 | 0.09 |
| Sample S2 | 9.1 | 0.09 |
| Sample S3 | 11.4 | 0.11 |
| Sample S4 | 17.4 | 0.17 |
| Sample S5 | 40.6 | 0.40 |

### <Prolapse Test>

### (Preparation of Example Guide Wire)

Guide wires of Examples 1 to 7 according to the present disclosure were prepared from the sample guide wire S described above. Specifically, seven types of guide wires were prepared for Examples 1 to 7, in which their distal and proximal end sides bordering on the position located 5 mm apart from the distal end of the sample guide wire S were applied with various coatings. The coating states on the distal end side with respect to the boundary, and the coating states on the proximal end side with respect to the boundary in each of Examples 1 to 7 are presented in Table 4.

**[Table 4]**

| | Distal end side | | Proximal end side | |
|---|---|---|---|---|
| | Coating state | Frictional resistance value (N) | Coating state | Frictional resistance value (N) |
| Example 1 | No coating | 0.40 | Silicone coating | 0.17 |
| Example 2 | Silicone coating | 0.17 | Silicone coating | 0.17 |
| Example 3 | Silicone coating | 0.17 | Hydrophilic coating (Film thickness: small) | 0.11 |
| Example 4 | Silicone coating | 0.17 | Hydrophilic coating (Film thickness: medium) | 0.09 |
| Example 5 | Hydrophilic coating (Film thickness: small) | 0.11 | Hydrophilic coating (Film thickness: medium) | 0.09 |
| Example 6 | Hydrophilic coating (Film thickness: small) | 0.11 | Hydrophilic coating (Film thickness: large) | 0.09 |
| Example 7 | Hydrophilic coating (Film thickness: medium) | 0.09 | Hydrophilic coating (Film thickness: medium) | 0.09 |

| | | | | |
|---|---|---|---|---|
| * The frictional resistance values are values under the test load of 1.47 N (150 gf). | | | | |

The "Silicone coating" in Examples 1 to 4 is the same coating layer as the silicone coating layer formed on the above-described sample S4. The "Hydrophilic coating (film thickness: large)" in Example 6 is the same coating layer as the hydrophilic coating layer formed on the above-described sample S1. The "Hydrophilic coating (film thickness: medium)" in Examples 4, 5, and 7 is the same coating layer as the hydrophilic coating layer formed on the above-described sample S2. The "Hydrophilic coating (film thickness: small)" in Examples 3, 5, and 6 is the same coating layer as the hydrophilic coating layer formed on the above-described sample S3. The "No coating" in Example 1 means that the sample is not coated as for the above-described sample S5. Thus, the surface frictional resistance values on the distal end side and the proximal end side of the guide wires according to Examples 1 to 7 are as presented in Tables 2 and 3.

### (Prolapse Test 1)

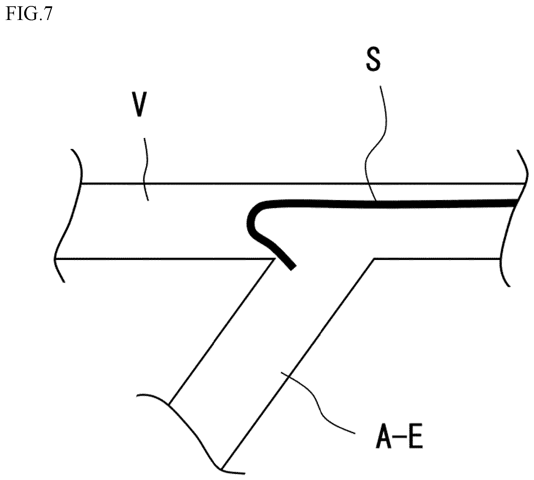

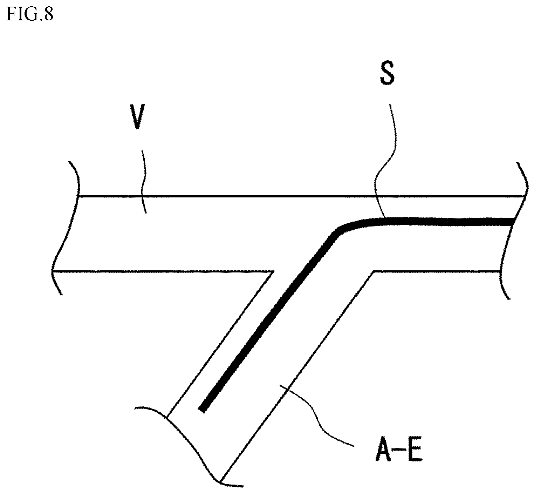

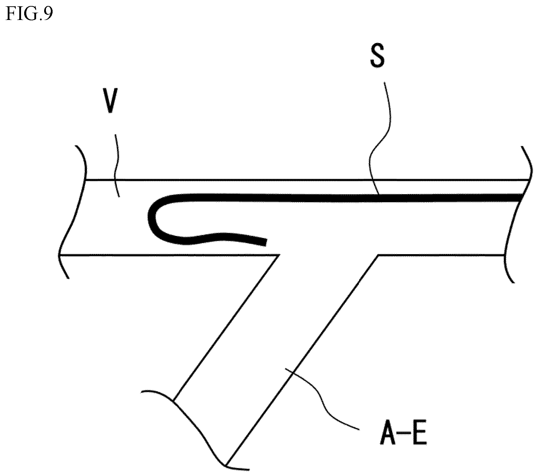

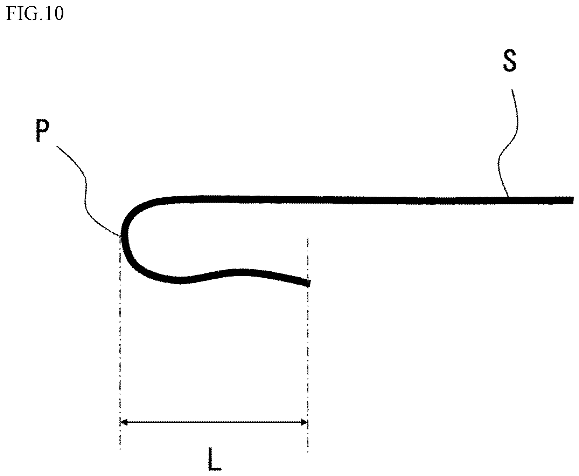

The distal end of the guide wire according to Examples 1 to 7 was shaped, and the guide wire was subjected to a test for confirming whether or not prolapse occurred when the guide wire was inserted, from its distal end, into a silicone blood vessel model M illustrated in FIG. 6 while the distal end of the guide wire was slightly shaped as if a surgeon handled a guide wire. The blood vessel model M has five lateral branches A, B, C, D, and E branching off from a middle of a main vessel V, and the lateral branches A to E are formed such that their connection angles θ with the main vessel V gradually increase in this order. An angle θA between the lateral branch A and the main vessel V is 15 degrees, an angle θB between the lateral branch B and the main vessel V is 30 degrees, an angle θC between the lateral branch C and the main vessel V is 45 degrees, an angle θD between the lateral branch D and the main vessel V is 60 degrees, and an angle θE between the lateral branch E and the main vessel V is 90 degrees. The main vessel V has an inner diameter of 3.0 mm, and the lateral branches A, B, C, D, and E have an inner diameter of 1.5 mm. Specifically, this blood vessel model was subjected to a test in which each of the guide wires according to Examples 1 to 7 was inserted, from its distal end, into the proximal end side (right side in FIG. 6) of the blood vessel model, and the distal end of the guide wire was intentionally hooked at each of the lateral branches A to E (FIG. 7), and in this state, it was confirmed whether the distal end of the guide wire entered the lateral branch (FIG. 8) or did not enter the lateral branch but prolapsed and progressed in the main vessel V (FIG. 9). The test results are presented in Table 5. FIG. 10 illustrates an enlarged view of the distal end portion of the prolapsing guide wire. In FIG. 10, L represents a progression length of the prolapse, and P represents a starting point of the prolapse.

**[Table 5]**

| | Lateral branch A | Lateral branch B | Lateral branch C | Lateral branch D | Lateral branch E |
|---|---|---|---|---|---|
| Example 1 | OK | OK | OK | OK | OK |
| Example 2 | OK | OK | OK | OK | OK |
| Example 3 | OK | OK | OK | OK | OK |
| Example 4 | OK | OK | OK | OK | OK |
| Example 5 | OK | OK | NG | NG | NG |
| Example 6 | OK | OK | NG | NG | NG |
| Example 7 | OK | OK | OK | NG | NG |

| | | | | | |
|---|---|---|---|---|---|
| OK ... The guide wire prolapsed and progressed in the main vessel V. NG ... The guide wire progressed in the lateral branch. | | | | | |

The above results show that prolapse occurs as long as the surface frictional resistance value of the guide wire on the distal end side is a certain level or higher. Particularly, in the guide wires according to Examples 1, 2, 3, and 4 with the frictional resistance value of 0.17 N or higher in a friction and wear test under a test load of 1.47 N and at a test rate of 120 mm/min (0.23 N or higher in a friction and wear test under a test load of 1.96 N and at a test rate of 120 mm/min), it was found that, even if the lateral branch had a large connection angle θ with the main vessel V, the distal end of the guide wire did not enter the lateral branch but prolapsed and progressed in the main vessel V. That means, as long as the surface frictional resistance value of the guide wire on the distal end side is 0.17 N or higher in a friction and wear test under a test load of 1.47 N and at a test rate of 120 mm/min (0.23 N or higher in a friction and wear test under a test load of 1.96 N and at a test rate of 120 mm/min), the prolapse is properly initiated. If the frictional resistance value is lower than the above-described value, the prolapse is not initiated even if the distal end is hooked, in some conditions of the lateral branch.

### (Prolapse Test 2)

Subsequently, the guide wires according to Examples 1 to 4 were subjected to a test for confirming how the progression length of the prolapse was changed when the guide wire was inserted into the main vessel V of the silicone blood vessel model illustrated in FIG. 5 while the distal end of the guide wire was bent by about 30 mm from the starting point and the guide wire was repeatedly pushed and pulled. The test results are presented in Table 6.

**[Table 6]**

| | Progression length of prolapse during insertion into main vessel V | Progression length of prolapse after repeated pushing and pulling |
|---|---|---|
| Example 1 | 30 mm | 20 to 30 mm |
| Example 2 | 30 mm | 20 to 30 mm |
| Example 3 | 30 mm | 5 to 10 mm |
| Example 4 | 30 mm | 5 to 10 mm |

The above results show that, even if excessive prolapse occurs (even if the progression length of the prolapse is larger than expected), as long as the surface frictional resistance value of the guide wire on the proximal end side is a certain level or lower, the prolapse does not progress over the certain level, so that the progression of the prolapse can be terminated. It is found that, by pulling the guide wire to return it to the proximal end side, the progression length of the prolapse can be restored to the progression length smaller than the progression length in the insertion. In particular, it was found that the progression of the prolapse could be returned to the vicinity of the boundary (5 mm apart from the distal end) in the guide wires in Examples 3 and 4 where the frictional resistance value was 0.11 N or lower in the friction and wear test under a test load of 1.47 N and at a test rate of 120 mm/min (0.18 N or lower in the friction and wear test under a test load of 1.96 N and at a test rate of 120 mm/min). That means, as long as the frictional resistance value of the proximal end-side surface of the guide wire is 0.11 N or lower in the friction and wear test under a test load of 1.47 N and at a test rate of 120 mm/min (0.18 N or lower in the friction and wear test under a test load of 1.96 N and at a test rate of 120 mm/min), the progression length of the prolapse can be appropriately controlled. If the frictional resistance value is higher than the above value, the progression of the generated prolapse may not terminate under some conditions of the blood vessel wall.

### DESCRIPTION OF REFERENCE NUMERALS

10, 10A: Guide wire
10a, 10Aa: First region
10b, 10Ab: Second region
1: Core shaft
11: Small diameter portion
12: Tapered portion
13: Large diameter portion
2: Cylindrical body
21: First surface region
22: Second surface region
3: Distal tip
4: Fixation portion
5a, 5b, 5c: Joint part
6: First covering layer
7: Second covering layer
8: Inner cylindrical body

## Claims

1. A medical device comprising:
a first region having a first surface property; and
a second region having a second surface property and located on a proximal end side with respect to the first region.

2. The medical device according to claim 1, wherein
the first surface property comprises a property that a surface of the first region has a first frictional resistance value in a friction and wear test performed on the surface of the first region,
the second surface property comprises a property that a surface of the second region has a second frictional resistance value in a friction and wear test performed on the surface of the second region under the same conditions as for the surface of the first region, and
the first frictional resistance value is higher than the second frictional resistance value.

3. The medical device according to claim 2, wherein when the friction and wear test is conducted under a test load of 1.96 N and at a test rate of 120 mm/min, the first frictional resistance value is 0.23 N or higher.

4. The medical device according to claim 2 or 3, wherein when the friction and wear test is conducted under a test load of 1.96 N and at a test rate of 120 mm/min, the second frictional resistance value is 0.18 N or lower.

5. The medical device according to any one of claims 2 to 4, wherein when the friction and wear test is conducted under a test load of 1.96 N and at a test rate of 120 mm/min, a difference between the first frictional resistance value and the second frictional resistance value is 0. 04 N or larger.

6. The medical device according to any one of claims 2 to 5, wherein when the friction and wear test is conducted under a test load of 1.96 N and at a test rate of 120 mm/min, a value obtained by dividing the first frictional resistance value by the second frictional resistance value is 1.2 or higher.

7. The medical device according to any one of claims 2 to 6, wherein when the friction and wear test is conducted under a test load of 1.47 N and at a test rate of 120 mm/min, the first frictional resistance value is 0.17 N or higher.

8. The medical device according to any one of claims 2 to 7, wherein when the friction and wear test is conducted under a test load of 1.47 N and at a test rate of 120 mm/min, the second frictional resistance value is 0.11 N or lower.

9. The medical device according to any one of claims 2 to 8, wherein when the friction and wear test is conducted under a test load of 1.47 N and at a test rate of 120 mm/min, a difference between the first frictional resistance value and the second frictional resistance value is 0. 05 N or larger.

10. The medical device according to any one of claims 2 to 9, wherein when the friction and wear test is conducted under a test load of 1.47 N and at a test rate of 120 mm/min, a value obtained by dividing the first frictional resistance value by the second frictional resistance value is 1.5 or higher.

11. The medical device according to claim 1, wherein
the first surface property comprises a property that a surface friction coefficient of the first region is a first friction coefficient,
the second surface property comprises a property that a surface friction coefficient of the second region is a second friction coefficient, and
the first friction coefficient is larger than the second friction coefficient.

12. The medical device according to claim 11, wherein the first friction coefficient is 0.1 or larger.

13. The medical device according to claim 11 or 12, wherein the second friction coefficient is 0.09 or smaller.

14. The medical device according to any one of claims 11 to 13, wherein a difference between the first friction coefficient and the second friction coefficient is 0.01 or larger.

15. The medical device according to any one of claims 11 to 14, wherein the value obtained by dividing the first friction coefficient by the second friction coefficient is 1.1 or higher.

16. The medical device according to any one of claims 1 to 15, wherein the first surface property comprises a property that the surface of the first region is hydrophobic.

17. The medical device according to any one of claims 1 to 16, wherein the first surface property comprises a property that a silicone coating layer is formed on the surface of the first region.

18. The medical device according to any one of claims 1 to 17, wherein the second surface property comprises a property that the surface of the second region is hydrophilic.

19. The medical device according to any one of claims 1 to 18, wherein the second surface property comprises a property that a hydrophilic coating layer is formed on the surface of the first region.

20. The medical device according to any one of claims 1 to 19, wherein the first region comprises a distal end of the medical device.

21. The medical device according to any one of claims 1 to 20, comprising:
an elongated core; and
a cylindrical body provided outside the core, wherein
an outer peripheral surface of the cylindrical body is partitioned into a first surface region located on a distal end side and a second surface region located on the proximal end side with respect to the first surface region.
